# EUROPEAN PATENT APPLICATION

(11) **EP 3 646 889 A1**
(43) Date of publication of application: **06.05.2020**
(21) Application number: 18382765.8
(22) Date of filing: 30.10.2018
(51) Int. Cl.: A61K 49/18

(54) **IRON OXIDE NANOPARTICLES DOPED WITH COPPER**

(71) Applicant: Centro Nacional de Investigaciones Cardiovasculares Carlos III (F.S.P.), 28029 Madrid (ES)
(72) Inventor: HERRANZ RABANAL, Fernando, 28029 Madrid (ES); FERNÁNDEZ BARAHONA, Irene, 28029 Madrid (ES)
(74) Representative: Hoffmann Eitle

(57) **Abstract**

In the present invention, we have found when iron oxide nanoparticles are doped with Cu (copper), a significant increase in the value of r1 is achieved (see figure 7), a value which measures the capacity of a contrast agent to provide a bright signal. "Normal" values of contrast agents for clinical use have values in the 4-6 range. In contrast, Cu-doped iron oxide nanoparticles (CuIONP), provide values of 16 mM⁻¹s⁻¹.

## Description

### Technical field of the invention

The present invention refers to the medical field, in particular to the field of medical diagnosis, more in particular to the use of iron oxide nanoparticles doped with copper for medical diagnosis and therapy.

### Background of the invention

In magnetic resonance imaging (MRI), the two main types of contrast that can be generated are based either in the reduction of water protons transverse relaxation time (T2) or the longitudinal relaxation time (T1). The first approach produces a darkening of the image, where the probe has accumulated, while the second renders a brightening of the imaging. For technical and physiological reasons, a T1-based contrast is preferred by radiologists and physicians for diagnosis. From the imaging point of view, this is a key aspect, being one of the main reasons why "traditional" IONP, for T2-weighted imaging, have not been established as an alternative in the clinical practice. The search for new iron oxide nanoparticle-based probes for magnetic resonance imaging (MRI) has traditionally focused on getting values of their transverse relaxivity (r2) as large as possible. However, in the last years the attention has moved to the development of iron oxide nanoparticles (IONP) as "positive" contrast agents, showing large values of their longitudinal relaxivity (r1). The rationale behind this approach is trying to get the best of the most commonly used contrast agents in MRI, Gd chelates, and the best of using nanoparticles. In other words, having a good, easy to spot, signal in MRI without a noticeable toxicity and the possibility of easily tuning the pharmacokinetic of the probe. There are two key aspects to have a good T1-based contrast agent: a large r1 value and a small r2/r1 ratio. The most common approach to obtain IONP as T1 contrast agents is by reducing the core size. Due to the large surface effects, IONP become more paramagnetic than superparamagnetic and the inner-sphere relaxation mechanism of water protons become more and more important. Alternatively, it is possible to modify the chemistry of the coating molecules on the nanoparticle to obtain T1-weighted contrast out of IONP in vivo. A third option is the core doping of the IONP with different metals. Even though there are few examples with this last option it is possible to find several to increase the r2 values (using Co, Ni, Zn or combinations of them), and also for positive contrast (mainly with Mn or Gd as the doping metals). As regards the use of Cu, there are examples where Cu doping effect is studied in catalysis, sensors performance, and in the magnetic properties of a nanomaterial. In the case of molecular imaging its use is quite limited, with one example, in combination with Co, using nanorods for MRI, and one example where 64Cu isotope is used for positron emission tomography. Interestingly in both cases the relaxometric properties of the nanomaterials make them only suitable for T2-weighted MRI (dark contrast). Furthermore, there are no studies on the use of Cu doping as a way to obtain nanoparticles for positive contrast MRI. The use of microwave-driven synthesis (MWS) for the production of nanomaterials is a versatile and highly reproducible approach. It has already been demonstrated how this methodology can produce, in extremely short times, homogeneous nanoparticles with excellent properties for molecular imaging. However, in the production of nanoparticles for T1-weighted MRI the use of MWS is far from common. Most of these syntheses are characterized by time-consuming protocols, multi-step approaches or the use of organic solvents.

Here, we have studied the effect of Cu doping iron oxide nanoparticles on their relaxometric properties. In a 10 minutes one-pot synthesis we have produced Cu core-doped IONP (CuIONP) with the largest longitudinal relaxivity value described so far, at clinically relevant magnetic fields. We have fully characterized the new nanomaterial and demonstrated its *in vivo* use for the enhanced diagnosis of tumors in animal models.

### Brief description of the invention

The majority of nanoparticles (NP) based on iron oxide provide for a negative (T2) signal which results in the area where these NPs accumulate to appear in a black colour. For several reasons, such colour complicates the diagnosis and thus limits its clinical use. For this reason research is searching for NPs capable of providing a positive bright T1 signal.

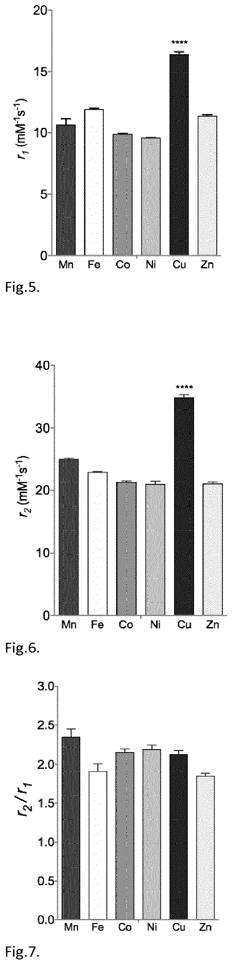

In this sense, we have found that when iron oxide nanoparticles are doped with Cu (copper), a significant increase in the value of r1 is achieved (see figure 7), a value which measures the capacity of a contrast agent to provide a bright signal. "Normal" values of contrast agents for clinical use have values in the 4-6 range. In contrast, Cu-doped iron oxide nanoparticles (CuIONP), provide values of 16 mM⁻¹s⁻¹.

### Brief description of the figures

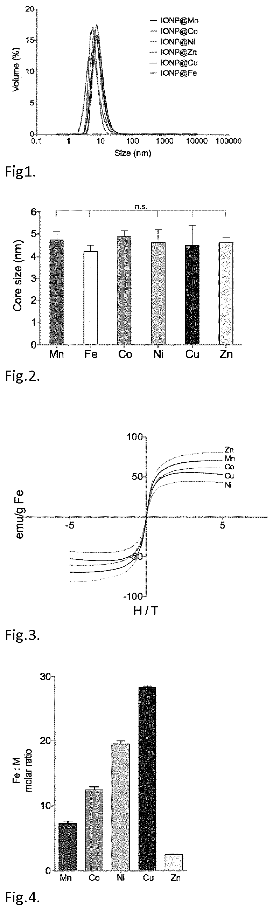

Fig1. Size of the synthesized particles, which have been doped with Mn, Co, Ni, Zn or Cu. Those indicated as IONP @ Fe are the original iron oxide, undoped. This figure indicates that the hydrodinamic size is the same for all.
Fig.2 Core size of all NPs shown in figure 1, with no differences between them.
Fig.3 Magnetism of the NPs shown in figure 1.
Fig.4 Molar ratio of Iron and each metal, indicating that those NPs doped with Cu are those that have less amount of dopant.
Fig.5 Values of r1, measure of how good the positive contrast is in MRI
Fig.6 Values of r2, measure of how good the negative contrast is in MRI, for any NP of iron oxide the r2 is always greater than the r1
Fig.7 Ratio r2 / r1, for any contrast agent, the higher the r1 and the lower the r2 / r1 ratio better contrast in T1. Cu NPs have an an r1 of 16 and r2 / r1 of 2.1.

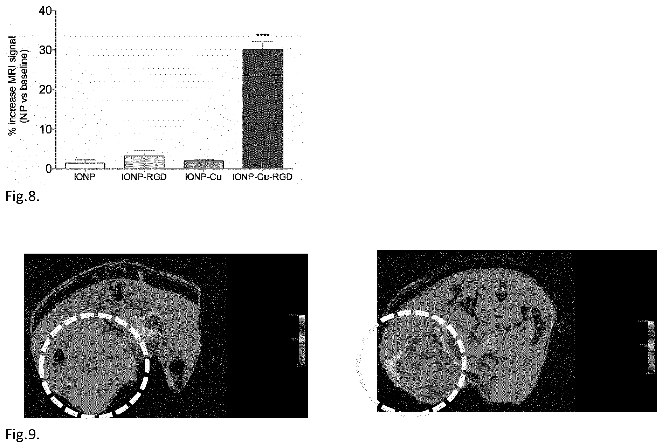

Fig.8 Percentage of increase in tumor signal in mice. IONP @ Fe (with and without peptide) and IONP @ Cu (with and without peptide) is compared
Fig.9. MRI of mice with tumors, before injecting NPs (left) and after (right). Tumor surrounded by the white circle.

### Detailed description of the invention

The authors of the present invention confronted with the problem of obtaining IONPs especially capable of providing a good contrast in *T1, synthesized particles doped with Mn, Co, Ni, Zn or Cu. For that purpose* they followed the synthetic procedure detailed in the examples provided herein. Interestingly, they found that when iron oxide nanoparticles are doped with Cu (copper), a significant increase in the value of r1 is achieved (see figure 7), a value which measures the capacity of a contrast agent to provide a bright signal. "Normal" values of contrast agents for clinical use have values in the 4-6 range. In contrast, Cu-doped iron oxide nanoparticles (CuIONP), provide values of 16 mM-1s-1. Therefore, these NPs are especially useful in the *in vivo* use for the enhanced diagnosis of tumors in human/animal models.

Thus, a first aspect of the invention refers to a preparation method of iron oxide nanoparticles (such as FeO, Fe₃O₄, Fe₄O₅, Fe₂O₃ or FeO₃, still more preferably the iron oxide is Fe₂O₃) doped with copper, comprising the following steps:
a. Preparing a mixture in water or an alcohol having a boiling point above 90°C, preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A copper salt such as CuCl₂ and an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃,
   b. Optionally, a biodegradable hydrophilic compound, wherein preferably the hydrophilic compound is a carboxylic acid, optionally selected from the list consisting of citric acid, malic acid, tartaric acid, and dimercaptosuccinic acid (DMSA),
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120ºC with microwave irradiation at 220-300W for 3-45 min, preferably, 5-45 min, more preferably 3-11 min; and
c. Purifying the nanoparticles obtained in step b).

A preferred embodiment of the first aspect of the invention refers to a preparation method of iron oxide nanoparticles (such as FeO, Fe₃O₄, Fe₄O₅, Fe₂O₃ or FeO₃, still more preferably the iron oxide is Fe₂O₃) doped with copper, comprising the following steps:
a. Preparing a mixture in water or an alcohol having a boiling point above 90ºC preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A copper salt and an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. Optionally, a biodegradable hydrophilic compound, wherein preferably the hydrophilic compound is a carboxylic acid, optionally selected from the list consisting of citric acid, malic acid, tartaric acid, and dimercaptosuccinic acid (DMSA) in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol; and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120ºC with microwave irradiation at 220-300W for 3-45 min, preferably, 5-45 min, more preferably 3-11 min; and
c. Purifying the nanoparticles obtained in step b).

A preferred embodiment of the first aspect of the invention refers to a preparation method of iron oxide nanoparticles (such as FeO, Fe₃O₄, Fe₄O₅, Fe₂O₃ or FeO₃, still more preferably the iron oxide is Fe₂O₃) doped with copper, comprising the following steps:
a. Preparing a mixture in water or an alcohol having a boiling point above 90ºC preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
   a. A copper salt and an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
   b. Optionally, a biodegradable hydrophilic compound, wherein preferably the hydrophilic compound is a carboxylic acid, optionally selected from the list consisting of citric acid, malic acid, tartaric acid, and dimercaptosuccinic acid (DMSA), in an amount of between 0.005 and 1 mmol, preferably in an amount of between 0.005 and 0.5 mmol, preferably in an amount of between 0.005 and 0.1 mmol, more preferably in an amount of between 0.005 and 0.03 mmol; and
   c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature of about 100ºC, with microwave irradiation at about 240W for about 10 min; and
c. Purifying the nanoparticles obtained in step b).

In a preferred embodiment of the first aspect of the invention, the purification step is carried out by gel filtration chromatography.

In the context of the present invention the term "about" is understood to indicate a value +/- 1% of the valued referred to therein.

In the context of the present invention the relaxivity (r1 or r2) of a contrast agent in water is the change in 1/T1 or 1/T2 of water per concentration of contrast agent.

In the context of the present invention a strong base is a basic chemical compound that can remove a proton (H+) from (or deprotonate) a molecule of a very weak acid in an acid-base reaction.

A second aspect of the invention refers to the nanoparticles obtained or obtainable by the preparation method of the first aspect of the invention or of any of its preferred embodiments.

On the other hand, a third aspect of the invention refers to an iron oxide nanoparticle, which comprises:
a. Optionally, a surface layer of a biodegradable hydrophilic carboxylic acid; and
b. A core comprising an iron oxide (such as FeO, Fe₃O₄, Fe₄O₅, Fe₂O₃ or FeO₃, still more preferably the iron oxide is Fe₂O₃), and copper;
wherein the nanoparticle is characterized by having the following values:
a. r1 is about 13 to 19 mM⁻¹s⁻¹, preferably about 16 as measured by relaxometry,
b. r2 is from 30-40 mM⁻¹s⁻¹ as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 20 +/-10 as measured by a Superconducting Quantum Interference Device (SQUID).

A preferred embodiment of the third aspect of the invention refers to an iron oxide nanoparticle doped with copper, which comprises:
a. A surface layer of a biodegradable hydrophilic compound, wherein preferably the hydrophilic compound is a carboxylic acid, optionally selected from the list consisting of citric acid, malic acid, tartaric acid, and dimercaptosuccinic acid (DMSA); and
b. A core comprising an iron oxide (such as FeO, Fe₃O₄, Fe₄O₅, Fe₂O₃ or FeO₃, still more preferably the iron oxide is Fe₂O₃), and copper,
wherein the nanoparticle is characterized by having the following values:
a. r1 is about 13 to 19 mM⁻¹s⁻¹, preferably about 16 as measured by relaxometry,
b. r2 is from 30-40 mM⁻¹s⁻¹ as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 20 +/-10 as measured by a Superconducting Quantum Interference Device (SQUID).

In addition and as already stated, the nanoparticles of the third aspect of the invention can be easily and reproducibly obtained by using the method detailed above.

The new nanoparticles and the preparation method described herein is very encouraging for clinical translation. Nanometric size iron oxides have already been approved by the FDA and other regulatory agencies. The nanoparticles described herein are produced in a reproducible and controlled manner, and, even with their dual modality character included, the present proposal facilitates Good Manufacturing Practice (GMP) compliance in approved production facilities required for clinical use.

Additionally, although this was not included in this work, we are not expecting safety or toxicological issues since the final formulations described herein include complete non-toxic components (iron oxide) and copper.

Thus a fourth aspect of the invention refers to the nanoparticles of the second, or third aspect of the invention for use as a medicament, or for use in therapy or medical diagnosis.

A fifth aspect of the invention refers to the nanoparticles of the second, or third aspect of the invention for use in a method of diagnosis and/or therapy capable of providing in vivo signals for positive contrast MRI (Magnetic Resonance Imaging).

Finally, a sixth aspect of the invention refers to the nanoparticles of the second, or third aspect of the invention for use in a method of diagnosis and/or therapy via systemic administration, preferably intravenous administration, capable of providing in vivo signals for positive contrast MRI (Magnetic Resonance Imaging).

The following examples have been inserted herein for illustration purposes only and thus do not limit the present invention.

### Examples

### EXPERIMENTAL SECTION

### Synthesis of IONP-citrate Samples

A mixture of FeCl₃·6H₂O (75 mg), citric acid trisodium salt (80 mg) was dissolved in water (9 mL). Subsequently, hydrazine monohydrate (1 mL) was added and the mixture was rapidly introduced into the microwave. Samples were heated under vigorous stirring at 240 W for 10 minutes at 120 °C. Once this step was completed, nanoparticles were purified through a gel filtration column (PD10) to eliminate unreacted species and stored in glass vials for further characterization.

### Synthesis of CuIONP-citrate Samples

A mixture of FeCl₃·6H₂O (75 mg), citric acid trisodium salt (80 mg) and CuCl₂ (2 mg, 6 mg, or 10 mg) was dissolved in water (9 mL). Subsequently, hydrazine monohydrate (1 mL) was added and the mixture was rapidly introduced into the microwave. Samples were heated under vigorous stirring at 240 W for 10 minutes at 120 °C. Once this step was completed, nanoparticles were purified through a gel filtration column (PD10) to eliminate unreacted species and stored in glass vials for further characterization.

### Synthesis of IONP-RGD

*To perform in vivo imaging in mice with tumor xenografts, RGD was covalently attached to carboxyl groups present in the citrate coating by EDC*/*sulfoNHS chemistry, yielding IONP-RGD. 12 mg of 1-ethyl-3-(3-dimethylaminopropyl)carbodiimide hydrochloride (EDC) and 15 mg of N-hydroxysulfosuccinimide (NHS) were added to 5 mL of IONP. Mixture was stirred at room temperature for 30 min and posteriorly purified by ultrafiltration using 30kDa cut-off filters. Filtered nanoparticles were diluted in buffer HEPES pH 8 to 1.8 mL and 1 mg of cycloRGD was then added and mixture stirred of room temperature for 60 min. Once this step was completed, sample was purified by ultrafiltration and resuspended in saline.*

### Synthesis of CuIONP-RGD

To perform in vivo imaging in mice with tumor xenografts, RGD was covalently attached to carboxyl groups present in the citrate coating by EDC/sulfoNHS chemistry, yielding CuIONP-RGD. 12 mg of 1-ethyl-3-(3-dimethylaminopropyl)carbodiimide hydrochloride (EDC) and 15 mg of N-hydroxysulfosuccinimide (NHS) were added to 5 mL of CuIONP. Mixture was stirred at room temperature for 30 min and posteriorly purified by ultrafiltration using 30kDa cut-off filters. Filtered nanoparticles were diluted in buffer HEPES pH 8 to 1.8 mL and 1 mg of cycloRGD was then added and mixture stirred at room temperature for 60 min. Once this step was completed, sample was purified by ultrafiltration and resuspended in saline.

### IONP and CuIONP Hydrodynamic Size and Zeta Potential Measurements

Hydrodynamic size and zeta potential of samples were measured using dynamic light scattering (DLS). A Nano Zetasizer ZS (Malvern Instruments) was used to this end. This device is equipped with a He-Ne laser operating at 633 nm and 4 mW and an avalanche photodiode detector.

### MRI Relaxation Properties of IONP and CuIONP

Relaxometric properties of the samples were assessed measuring longitudinal and transverse relaxation times. Four concentrations of each nanoparticle sample were selected and longitudinal and transversal relaxation times of each one measured in a Bruker Minispec mq60 contrast agent analyzer at 1.5 T and 37 °C. r₁ and r₂ values were plotted against the Fe concentration (0, 0.25, 0.5, 1, and 2 mM).

### Hydrodynamic Size and Zeta Potential Measurements

Hydrodynamic size and zeta potential of samples were measured using dynamic light scvattering (DLS). A Nano Zetasizer ZS (Malvern Instruments) was used to this end. This device is equipped with a He-Ne laser operating at 633 nm and 4 mW and an avalanche photodiode detector.

### In vivo MRI Acquisition

In vivo MRI in mice was performed with an Agilent/Varian scanner (Agilent, Santa Clara, CA, USA) equipped with a DD2 console and an active-shielded 205/120 gradient insert coil with 130 mT m-1 maximum gradient strength and a combination of a volume coil and a 2-channel phased array (Rapid Biomedical GmbH, Rimpar, Germany).

## Claims

1. An iron oxide nanoparticle which comprises a core comprising an iron oxide and copper; wherein the nanoparticle is **characterized by** having the following values:
a. r1 is about 13 to 19 mM⁻¹s⁻¹, preferably about 16 as measured by relaxometry,
b. r2 is from 30-40 mM⁻¹s⁻¹ as measured by relaxometry,
c. a core size of from 1.5 - 3 nm as measured by Transmission Electron Microscopy (TEM),
d. a hydrodynamic size of from 20 ± 5 nm according to dynamic light scattering measurement (DLS), and
e. a superparamagnetic behavior with a value of magnetic saturation (Ms) of from 20 +/-10 as measured by a Superconducting Quantum Interference Device (SQUID).

2. The iron oxide nanoparticle of claim 1, wherein the iron oxide is selected from the group consisting of FeO, Fe₃O₄, Fe₄O₅, Fe₂O₃ or FeO₃.

3. An iron oxide nanoparticle which comprises:
a. A surface layer of a biodegradable hydrophilic compound; and
b. A core comprising an iron oxide selected from the group consisting of FeO, Fe₃O₄, Fe₄O₅, Fe₂O₃ or FeO₃, and copper;
wherein the nanoparticle is **characterized by** having the values defined in claim 1.

4. A preparation method of iron oxide nanoparticles (such as FeO, Fe₃O₄, Fe₄O₅, Fe₂O₃ or FeO₃, still more preferably the iron oxide is Fe₂O₃) doped with copper, comprising the following steps:
a. Preparing a mixture in water or an alcohol having a boiling point above 90ºC preferably selected from the group consisting of benzyl alcohol, ethanol, Isopropyl alcohol, ethylene glycol, 1,3-propanediol, 1,4-butanediol, glycerol and octanol, having a pH between 8 and 12, comprising the combination of
a. A copper salt and an iron salt preferably selected from the group consisting of FeCl₃, Fe(SO4), Fe₂(SO4)₃, Fe(NO₃)₃, FeCO₅, Fe(OH)₂ and Fe(OH)₃, more preferably FeCl₃, in an amount of between 0.05 and 110 mmol, preferably in an amount of between 0.1 and 10 mmol, preferably in an amount of between 0.1 and 1mmol, more preferably in an amount of between 0.1 and 0.5 mmol;
b. Optionally, a biodegradable hydrophilic compound, wherein preferably the hydrophilic compound is a carboxylic acid, optionally selected from the list consisting of citric acid, malic acid, tartaric acid, and dimercaptosuccinic acid (DMSA), preferably in an amount of between 0.005 and 0.5 mmol, preferably in an amount of between 0.005 and 0.1 mmol, more preferably in an amount of between 0.005 and 0.03 mmol; and
c. a strong base in a sufficient amount for the mixture to have a pH between 8 and 12, wherein preferably said strong base is Hydrazine hydrate,
b. Subjecting said mixture to fast ramping (in less than three minutes, preferably in less than 1 minute) to a temperature between 90 and 120ºC with microwave irradiation at 220-300W for 3-45 min, preferably, 5-45 min, more preferably 3-11 min; and
c. Purifying the nanoparticles obtained in step b).

5. The preparation method of iron oxide nanoparticles of claim 4, wherein the iron salt is selected from the group consisting of FeCl₃, Fe(SO4), Fe₂(SO4)₃, Fe(NO₃)₃, FeCO₅, Fe(OH)₂ and Fe(OH)₃.

6. The preparation method of iron oxide nanoparticles of any of claims 4 to 5, wherein the mixture of step a) is subjected in step b) to fast ramping in less than one minute to about 100 ºC with microwave irradiation at 220-240W for 8-11 min.

7. The preparation method of iron oxide nanoparticles of any of claims 4 to 5, wherein the mixture of step a) is subjected in step b) to fast ramping in less than one minute to about 100 ºC with microwave irradiation at about 240W for about 10 min.

8. A nanoparticle as defined in any of claims 1-3 for use in therapy or medical diagnosis.

9. A nanoparticle as defined in any of claims 1-3 for use in a method of diagnosis and/or therapy capable of providing positive contrast MRI (Magnetic Resonance Imaging).
